(19) Europäisches Patentamt
European Patent Office
Office européen des brevets

(11) **EP 4 585 139 A1**

(12) **EUROPEAN PATENT APPLICATION**

(43) Date of publication:
**16.07.2025  Bulletin 2025/29**

(21) Application number: **24382030.5**

(22) Date of filing: **12.01.2024**

(51) International Patent Classification (IPC):
**A61B 3/00** (2006.01)          **A61B 3/10** (2006.01)
**A61B 3/103** (2006.01)

(52) Cooperative Patent Classification (CPC):
**A61B 3/0025; A61B 3/1015; A61B 3/103**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Fundacion Instituto De Investigacion Sanitaria Fundacion Jimenez Diaz 28040 Madrid (ES)**

(72) Inventors:
• **FARIA RIBEIRO, Miguel António Braga (PT)**
• **VELARDE RODRÍGUEZ, Gonzalo Rafael 28040 Madrid (ES)**
• **ALEJANDRE ALBA, Nicolás 28040 Madrid (ES)**
• **JIMÉNEZ-ALFARO MOROTE, Ignacio 28040 Madrid (ES)**

(74) Representative: **Hoffmann Eitle Hoffmann Eitle S.L.U. Paseo de la Castellana 140, 3a planta Edificio LIMA 28046 Madrid (ES)**

(54) **METHOD FOR DETERMINING A REFRACTIVE CORRECTION FOR AN OPTICAL SYSTEM**

(57)     The present disclosure relates to a computer-implemented method for determining a refractive correction in an optical system, such as an eye. This method involves the utilization of data representing a light wavefront that has passed through the optical system to compute a curvature map. The curvature map is then segmented into at least two clusters based on the variation between the curvature data points. For each cluster, the mean curvature of the light wavefront is calculated. Subsequently, the refractive correction corresponding to the mean curvature of the light wavefront in each cluster is computed. The final step involves selecting one of these computed refractive corrections as the definitive refractive correction for the entire optical system. This method allows for a quick and precise prescription determination for any type of optical correction.

EP 4 585 139 A1

## Description

## Technical field of the invention

The present invention relates to the field of optical diagnostics and vision correction technology. Specifically, it relates to computer-assisted methods for analysing and determining refractive errors in optical systems. More precisely, this invention is a computer-implemented method for determining a refractive correction for an optical system such as the human eye.

## Background of the invention

The human eye functions as a complex optical system, finely tuned to focus on objects across various distances. This system primarily consists of two optical elements: the cornea and the crystalline lens. The cornea, the eye's foremost part, is responsible for about two-thirds of its total refractive power, playing a pivotal role in directing light towards the retina. Meanwhile, the crystalline lens, a flexible biconvex lens situated behind the cornea, contributes approximately one-third of the eye's focusing ability. This lens is especially crucial for accommodation, the process of adjusting focus to perceive near objects clearly. An eye called emmetrope is one whose refractive power and axial length are adjusted so that a beam of parallel rays, coming from an object at infinity, forms a clear image on the retina without the need to accommodate. Any imperfection in the geometry of these optical components or an unsuccessful combination between focal power and axial length results in a refractive error, with a consequent impact on the quality of the image perceived by that subject. Uncorrected refractive errors are the most common cause of reversible vision impairment in the modern world.

Incidentally, clinical assessment of the refractive status of the eye is a common task in the clinical practice of optometry and ophthalmology. The standard technique for evaluating the refractive status, called the subjective method, consists of a sequential strategy with the objective of finding the best combination of spherical and cylindrical lenses that produce the best retinal image quality, according to the subjective evaluation criteria of the patient and the visual task performed during this process. Normally, this sequential method is preceded by an objective method, such as retinoscopy, or autorefraction to establish a starting point closer to the desired one and, therefore, optimize the process of looking for the best possible combination of lenses. However, even so, the objective determination of the patient's refractive status presents important challenges, as it is often a time-consuming process of trial and error, and the patient's subjective evaluation can lead to local maxima of visual acuity that do not necessarily represent the optimal (or global maximum) correction. This makes it difficult to manually find an optimized solution for the patient's visual needs.

Objective refractive error determination, on the other hand, aims to avoid the subjective input from the patient and the trial and error related to traditional subjective methods. One of the major advances achieved in recent decades in the field of objective determination of the refractive status of the eye was the introduction of wavefront aberrometry into clinical practice. Modern wavefront aberrometers can produce a comprehensive description of the optical imperfections (aberrations) of the eye and display the result in the form of a descriptive map of the optical path variation, suffered by the light passing through each analysed point in the pupil.

However, the problem of determining an optimized visual prescription from the information provided by an aberrometer has proven more challenging than expected. To ascertain the prescription, or best correction, based on image quality metrics, it is necessary to solve a highly complex nonlinear optimization problem. This nonlinear search involves navigating a three-dimensional space comprising three unknown variables: sphere (S), cylinder (C), and axis (E0) - alternatively represented in vector form decomposition as M, CJ0, and J45. Nonlinear optimization in such a multidimensional space, however, is susceptible of converging towards local minima, and, furthermore, these methods typically require starting from an approximation that is reasonably close to the ultimate solution, or the global minimum. Moreover, there is an absence of a standardized criterion for objective refraction metrics and image quality metrics, and different image quality metrics may eventually give different results.

An objective method has been described to overcome these issues that combines linear calculus and linear algebra, and that provides a reasonable result in agreement with the best image quality metrics. This method uses a definition of vergence that calculates the vergence error in dioptres for each ray perpendicular to the wavefront as it exits the pupil. This error is represented by a 2x2 matrix, named V, which has three independent elements, directly related to the slopes of the wavefront: the spherical aberration S, Coma-like horizontal aberrations C0 and coma-like oblique aberrations C45. The V matrix represents the shape of the beam formed by each ray, and this conical beam shape (such as ellipse, hyperbola, etc.) may be defined by these three types of refractive errors: S, C0, and C45 (or, alternatively, mean spherical equivalent M, Jackson Cross Cylinder with the axis oriented at 0 and 90 degrees J0 and Jackson Cross Cylinder with the axis oriented at 45 and 135 degrees J45, since both nomenclatures refer to equivalent optical measurements or properties). Essentially, the beam created by each ray is similar to what would be produced by combining spherical and cylindrical lenses.

Although this approach is accurate, a crucial issue remains on how to determine the global refractive error from a set of individual samples. One approach consists of evaluating hundreds or thousands of prescriptions associated each to a point in the curvature map of the light wavefront, in order to find the best one according to a certain image quality metric. However, the number of calculations to evaluate such a big number of prescriptions make this method unfit for quick and *in situ* refractive error determination, as it is computationally very expensive. The proposed solutions so far use a simple statistical mean, or the statistical mode, over the calculated local refractive errors. However, this approach is greatly influenced by outliers, i.e. marginal values too big or too small, which have excessive statistical influence. Besides, it does not take into account that some parts of the cornea have a similar curvature, and a prescription adapted to this particular geometry, even though not representing the curvature of the whole pupil, represents a surface big enough to yield a much better visual acuity for that particular refractive correction than would a prescription adapted to a general average of very disparate points. Not only that, in the presence of higher order aberrations, such as spherical aberration, the previous methods can easily be biased by the larger area covered by the outer parts of the pupil, that usually do not form the most compact image of a point, i.e., do not provide the best retinal image quality. Additionally, as previously mentioned, analysing individually all prescriptions possible, each for every point measured with aberrometry, in order to find the best one, is computationally very expensive and takes too much time, making it unfit to use it in clinical practice in an ophthalmological centre during a patient's visit for an eye corrector prescription. Besides, even if all the local point prescriptions are evaluated, it may still not be comprised therein a value of a global prescription that optimizes the visual acuity for an eye of a patient as a whole, or as a big enough part of it.

Therefore, there is clearly a need of a method to obtain an objective optimized global visual prescription, that performs outstandingly good in the best image quality metrics, and that therefore is a good representation of a realistic subjective visual quality. Furthermore, this method should avoid giving a tipping importance to outlier points or areas from the wavefront data analysed, and it should not require starting from a close approximation to the final solution. Besides, this method must be computationally lightweight and quick, in order to be able to be carried out in seconds, such that it can be performed, for example, during a patient's ophthalmological visit, in order to provide the patient with a good prescription in situ, and not needing a second appointment delayed in time.

**Summary of the invention**

A first aspect of the invention refers to a computer implemented method for determining a refractive correction for an optical system, the method comprising the following steps:

    a. computing a curvature map from data representing a light wavefront that has passed through the optical system;

    b. segmenting the curvature map computed in step (a) into at least two clusters of curvature data points according to the variation between the data points within each cluster;

    c. computing the mean curvature of the light wavefront in each cluster from step (b);

    d. computing for each cluster the refractive correction associated to the mean curvature of the light wavefront of the cluster as computed in step (c); and

    e. determining the refractive correction for the optical system as one of the refractive corrections computed in step (d) of the clusters of the curvature map.

In a preferred embodiment of the method of the first aspect of the invention, in step b) the curvature map is segmented into at most 20 clusters.

In a more preferred embodiment of the method of the first aspect of the invention, in step b) the curvature map is segmented into at most 10 clusters.

In a preferred embodiment of the method of the first aspect of the invention, in step b) the curvature map is segmented into a predefined number of clusters.

In a preferred embodiment of the method of the first aspect of the invention, the number of clusters is determined using any of the following methods or any of their combinations: k-means method, inertia method, DBScan, Hierarchical Clustering, and spectral clustering.

In a preferred embodiment of the method of the first aspect of the invention, the at least two clusters are segmented according to a predefined maximum standard deviation between the data points within each cluster, preferably wherein the maximum standard deviation is 0.5 dioptres (D).

In a preferred embodiment of the method of the first aspect of the invention, the curvature map comprises one or more of the following: a mean curvature map (M), a cartesian astigmatism map (J0) and/or oblique astigmatism map (J45), preferably the curvature map comprises the mean curvature map (M).

In a preferred embodiment of the method of the first aspect of the invention, the optical system represents an eye which suffers from astigmatism, coma, spherical aberration and/or comprises an irregular cornea, an ectasia, and/or represents an eye which has undergone corneal surgery, and wherein the curvature map comprises the mean curvature map (M), the cartesian astigmatism map (J0) and the oblique astigmatism map (J45).

In a preferred embodiment of the method of the first aspect of the invention, the refractive correction is determined as the mean refractive correction of the cluster of the curvature map that yields the best image quality according to a predesignated image quality metric.

In a preferred embodiment of the method of the first aspect of the invention, the refractive correction is determined as the mean refractive correction of the cluster with the largest area and/or lowest variation between the data points within each cluster.

In a more preferred embodiment of the method of the first aspect of the invention, the best refractive correction is determined according to at least one image quality metric.

In an even more preferred embodiment of the method of the first aspect of the invention, the at least one image quality metric comprises one or more of the following metrics: Visual Strehl ratio based on the extended area (VSX), Visual Strehl ratio based on the modulation transfer function (VSMTF), Visual Strehl ratio that is based on the optical transfer function (VSOTF).

In a preferred embodiment of the method of the first aspect of the invention, the method further comprises determining the sphere, cylinder and axis of such refractive correction.

A second aspect of the invention refers to a computer implemented program comprising instructions which, when executed by a processing unit, configures the processing unit to execute the computer-implemented method according to any one of claims 1 to 12.

A third aspect of the invention refers to a system for determining a refractive correction, the system comprising: a processing unit and a memory unit, wherein the memory unit comprises a set of instructions configured to execute the computer-implemented method according to any one of claims 1 to 13, and wherein the processing unit is configured to execute such instructions from the memory unit.

**Brief description of the drawings**

To enable a better understanding of the present disclosure, and to show how the present disclosure may be carried out, reference will now be made, by way of example only, to the accompanying schematic drawings, wherein:

Figure 1 shows a "mean" (M) curvature map, or local spherical equivalent refractive error, extracted from data representing a light wavefront passing through a pupil or diaphragm, in dioptres.

Figure 2 shows the image acuity resolved by an imperfect optical system, such as an eye suffering from refractive errors, after a prescription, or optical correction, has been applied according to a method from the current state of the art that averages the refractive error correction from the map of figure 1.

Figure 3 shows a segmentation of the "mean" curvature map shown in figure 1, into 4 clusters according to one or more embodiments of the invention.

Figure 4 shows a table with the Sphere, Cilinder and Axis curvature values, along with the refractive error and the standard deviation for each cluster represented in figure 3, according to one or more embodiments of the invention.

Figure 5 shows the image acuity resolved by an imperfect optical system, such as an eye suffering from refractive errors, after a prescription, or optical correction, obtained from cluster 3 of figures 3 and 4, has been applied, according to one or more embodiments of the invention.

Figure 6 shows the wavefront error from a light wavefront passing through the pupil or diaphragm of an optical system, such as an eye.

Figure 7 shows the M (fig. 7 a1), J0 (fig. 7 b1) and J45 (fig. 7 c1) curvature maps, or local refractive errors, extracted from data representing a light wavefront passing through a pupil or diaphragm, in dioptres. Images a2, b2 and c2 are local refractive error frequency histograms corresponding to images a1, b1 and c1 respectively, in percentage.

Figure 8 a) shows an inertia measurement as a function of the number of clusters for the "mean" curvature map from figure 7 a1), wherein the global minimum is 6 clusters.

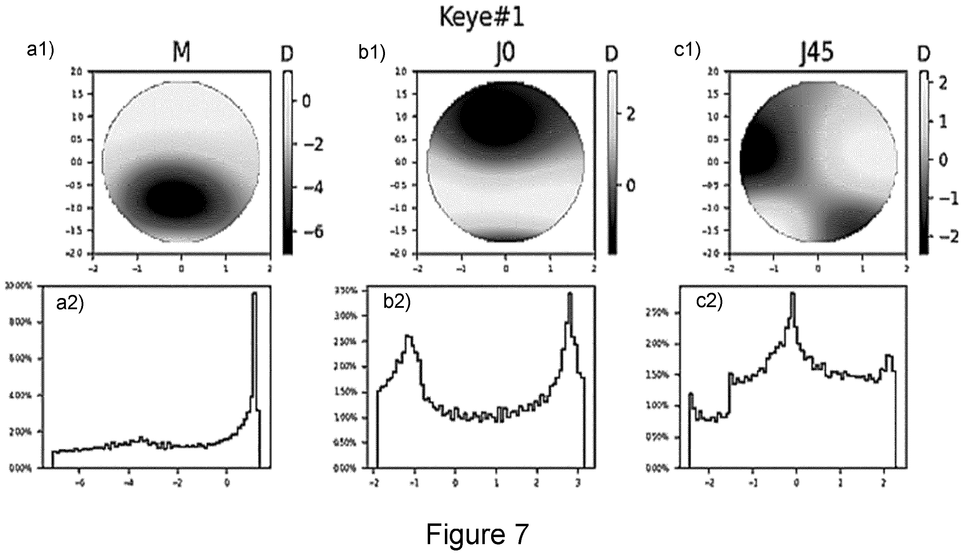

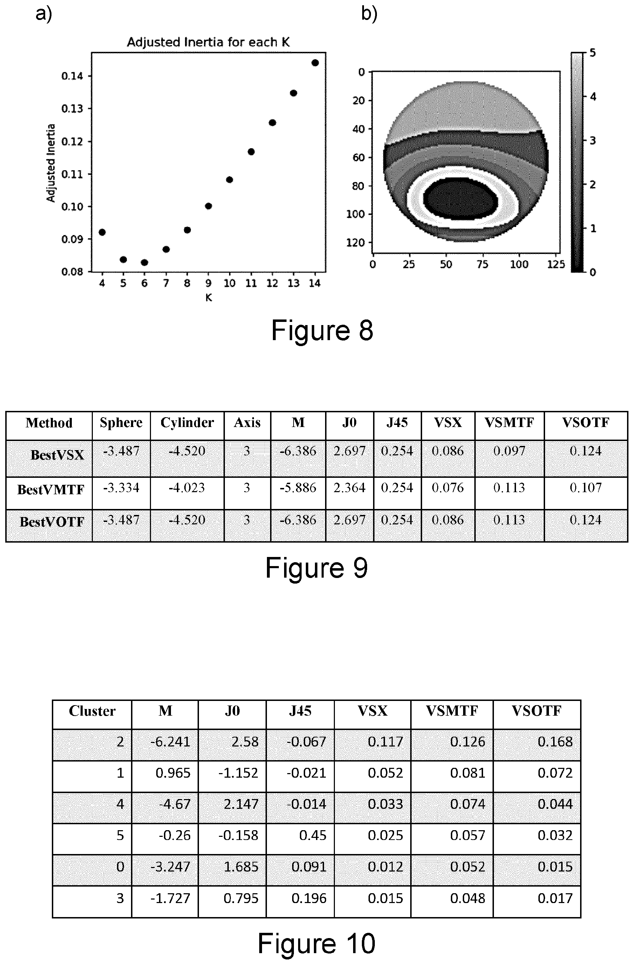

Figure 8 b) shows a k-means segmentation of the "mean" curvature map shown in figure 7 a1), into 6 clusters, in relation to the optimal number of clusters obtained in figure 8 a), according to one or more embodiments of the invention.

Figure 9 shows the prescriptions (Sphere, Cylinder and Axis) obtained from 648 "mean" (M), and J0 and J45 astigmatism curvature maps that yield the best, or optimal, image acuity according to three metrics that evaluate the quality of optical systems, namely VSX, VSMTF and VSOTF. The three metrics converge towards the same prescription and values.

Figure 10 shows a table with the optical quality metrics VSX, VSMTF and VSOTF values for the "mean" (M), and J0 and J45 astigmatism curvature maps of each of the six clusters obtained in figure 8b). Noticeably, cluster 2 presents a high similarity with the VSX, VSMTF and VSOTF values optimal values obtained from figure 9.

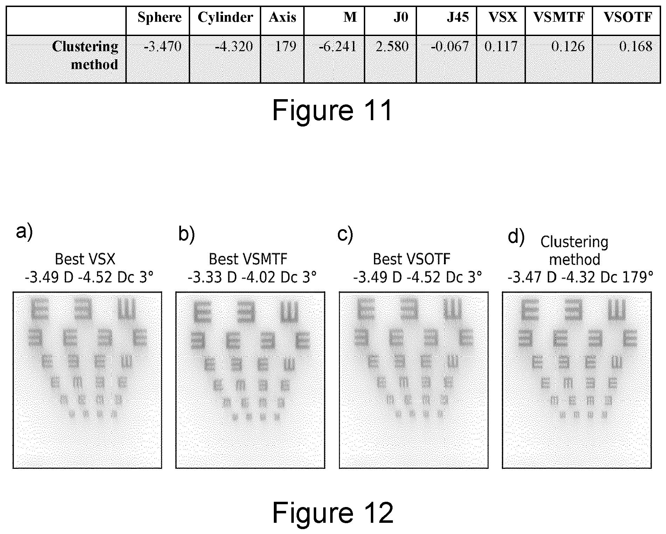

Figure 11 shows a table with the optical quality metrics VSX, VSMTF and VSOTF values for the "mean" (M), and J0 and J45 astigmatism curvature maps of cluster 2 from images 10 and 8b), it also shows the corresponding prescription (Sphere, Cylinder and Axis) in the plane of the pupil, according to one or more embodiments of the invention.

Figure 12 a), b) and c) shows the image acuity resolved by an imperfect optical system, such as an eye suffering from refractive errors, after the best prescription from figure 9 obtained according to the optical quality metrics VSX, VSMTF and VSOTF from the 648 prescriptions evaluated have been applied, respectively. Figure 12 d) shows the image acuity resolved after the prescription obtained according to the refractive error from cluster 2 from images 8b), 10 and 11 has been applied. Cluster 2 is selected because it has the best image quality according to the three metrics used in figure 12 a, b and c, respectively. It is noted that all 4 images (a, b, c, and d) are almost indistinguishable and show a strikingly similar visual acuity.

## Description of the invention

## Definitions

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20%, preferably +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of' excludes any element, step, or ingredient not specified in the claim element. When used herein, "consisting essentially of' does not exclude materials or steps that do not materially affect the basic and novel characteristics of the claim.

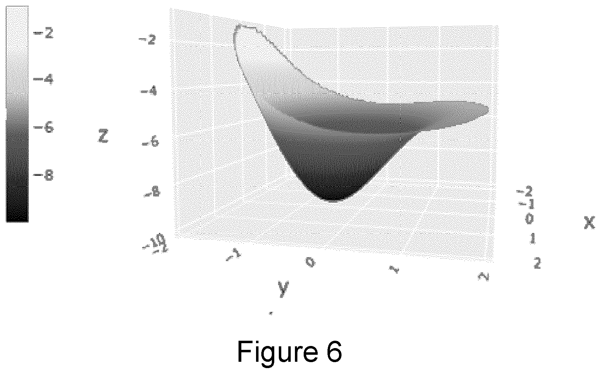

The term "light wavefront", in the context of the invention, refers to a surface over which the phase of the light wave is constant at a given instant in time. In a uniform medium, these wavefronts are typically spherical or planar, depending on the source of the light. In the context of the invention, the wavefronts are perfectly spherical when non-aberrated, whereas aberrated wavefronts, such as the ones from a visually impaired patient, deviate from this perfect spherical shape. Said wavefronts expand outward in all directions. Light wavefront may be measured with aberrometers, or it may be calculated using virtual eyes models, modelled using commercially available platforms such as Zemax or OSLO. Additionally, in the context of the invention, the light wavefront may be obtained with a combination of aberrometer and virtual eye models. A representation of the light wavefront error is depicted in figure 6, representing how much deviation the wavefront of the eye has compared to a perfect spherical reference wavefront. It is noted that because when measured with an aberrometer, the reference wavefront is flat (as it should emerge from the eye with zero curvature to focus on infinite), both the wavefront and wavefront error are the same in this case.

The term "refractive correction", in the context of the invention, refers to the adjustment made to an optical system, for instance to an eye, to correct its refractive error. This correction improves the focus of light onto the retina, thereby enhancing visual clarity. In the method of the invention, refractive correction is determined based on the calculated mean curvature of a light wavefront passing through the optical system. Preferably the refractive correction is represented by the parameters S, C0 and C45, wherein S is the spherical aberration, C0 is the coma-like horizontal aberrations and C45 is the coma-like oblique aberrations.

The term "pupil", in the context of the invention, refers not just to the pupil of the eye in its traditional sense as a diaphragm controlling light entry, but more specifically to the area of the cornea that corresponds to the pupil's location. This definition highlights the segment of the cornea directly aligned with the pupil, which plays a critical role in focusing light onto the retina. Preferably it refers to the pupillary area of the cornea when the pupil is dilated, or to the pupillary area in normal light conditions. Additionally, in broader optical systems, the term can extend to the area of a lens that corresponds to a diaphragm, such as the pupil in the human eye, playing a similar role in controlling and focusing light. Furthermore, in the scope of this invention, the concept of the pupil can also include a virtual or 3D simulated representation.

The term "optical system", in the context of the invention, refers to any arrangement of components that interact with light to affect its transmission, direction, or focus. In this invention, preferably it denotes the human eye, more preferably it is related to the pupil, or more precisely to the cornea and its curvature within the pupillary aera.

The term "variation between the data points", in the context of the invention, refers to the differences or discrepancies in the curvature values of the light wavefront data points as it passes through the optical system, like the human eye or a similar optical device. This variation may be measured using various mathematical methods for data deviation, such as, but not limited to, standard deviation, variance, range, or mean absolute deviation. These methods quantify the extent to which individual data points on the curvature map, representing specific curvature measurements at different corneal locations, deviate from a central value or from each other. This quantitative assessment of variation may be used for segmenting the curvature map into clusters with similar refractive characteristics.

The terms "k-means method, inertia method, DBScan, Hierarchical Clustering, and spectral clustering", in the context of the invention, refers to different clustering methods used in data analysis, particularly for segmenting the curvature map in the optical system. K-means method is an iterative algorithm that partitions the dataset into a predetermined number of clusters (k) by minimizing the variance within each cluster. Inertia method is often used in conjunction with k-means, and this method quantifies the compactness of clusters, helping to determine the optimal number of clusters by minimizing within-cluster distances. DBScan (Density-Based Spatial Clustering of Applications with Noise) identifies clusters based on the density of data points, proving useful in handling outliers and identifying arbitrarily shaped clusters in the curvature data. Hierarchical Clustering builds a hierarchy of clusters either by successively merging smaller clusters into larger ones (agglomerative approach) or by dividing a large cluster into smaller ones (divisive approach), suitable for detailed, multi-level clustering analysis. Finally, Spectral Clustering uses eigenvalues of a similarity matrix to reduce dimensionality before clustering, allowing for the identification of complex, non-linearly separable clusters in the curvature data.

The term "dioptre" refers to a unit of measurement for the optical power of a lens or curved mirror. It is defined as the reciprocal of the focal length measured in meters (in air). In the context of the invention, this term is used to describe the refractive power needed to correct vision.

The term "mean curvature of the light wavefront", in the context of the invention, refers a statistical representation of the curvature of the wavefront of light as it passes through an optical system, like the eye, preferably refers to the mathematical average. This measurement represents the general bending or shaping of the wavefront due to the refractive properties of the optical system. The mean curvature of the wavefront provides a quantitative representation of the refractive error in an optical system, which in the eye is a result of the deviation of light caused by the shape of the cornea and/or the lens, wherein a higher or lower than zero mean curvature indicates a refractive error, therefore the "mean curvature of the light wavefront" gives an indication of the local "mean refractive error".

The term "eye corrector", or optical corrector, in the context of the invention, refers to any device or procedure designed to adjust or compensate for the refractive errors of an eye, or optical system, thereby improving visual clarity. This term comprises eyeglasses, contact lenses, intraocular lenses, and various forms of refractive surgery, among others.

The term "mean curvature map (M)" refers to a specific component of refractive error analysis in power vector notation, which includes M, J0, and J45. In this context, "M" represents the spherical equivalent of refractive error, capturing the overall degree of myopia or hyperopia. The mean curvature map (M) visually depicts this spherical component, showing how the local average curvature of the eye's surface deviates from a perfectly spherical shape.

The term "cartesian astigmatism map (J0)" refers to a component of refractive error analysis, particularly focusing on astigmatism, in the power vector notation that includes M, J0, and J45. Specifically, J0 represents the degree of astigmatism oriented along the Cartesian axes - either horizontal or vertical. The cartesian astigmatism map (J0) visually portrays this type of astigmatism, indicating how the curvature of the eye's surface varies in the horizontal (180 degrees) and vertical (90 degrees) meridians, in each sampled point.

The term "oblique astigmatism map (J45)" refers to a specific component in the power vector analysis of refractive error, alongside M and J0. J45 represents the degree of astigmatism that is oriented along the oblique axes, at 45 and 135 degrees. The oblique astigmatism (J45) map visually illustrates this type of astigmatism, showing the variations in the curvature of the eye's surface along these oblique meridians, in each sampled point.

The terms "Sphere, Cylinder, and Axis" refer to standard parameters used in the prescription of corrective lenses for refractive errors in the eye. "Sphere" indicates the degree of myopia (near-sightedness) or hyperopia (far-sightedness) and is measured in dioptres, representing the lens power needed for correcting spherical refractive errors. "Cylinder" quantifies the degree of astigmatism, also in dioptres, showing how much lens power is required to correct the asymmetrical curvature of the eye. The "Axis" specifies the orientation of the astigmatism in degrees, indicating the direction of the cylindrical correction required. It is noted that it is known the conversion from M, J0 and J45 power vectors to "Sphere, Cylinder and Axis" lens power vectors.

The term "image quality metric" refers to a quantitative measure used to assess the quality of an image, particularly in the context of optical systems like cameras, telescopes, and the human eye. These metrics evaluate various aspects of image clarity, sharpness, contrast, and overall visual perception. Examples include, but are not limited to, VSX, VSMTF, VSOTF, MTF (Modulation transfer function), PSF (Point spread function), and Wavefront Error, among others.

The term "VSX", or Visual Strehl Ratio based on the extended area, refers to an image quality metric used to assess the performance of optical systems, including the human eye, particularly in the context of refractive corrections. Unlike the traditional Strehl ratio, which is based on the peak value of the Point Spread Function (PSF), VSX considers a more extended area of the PSF. This approach provides a more comprehensive evaluation of the optical quality, taking into account not just the peak performance but also how well the system performs over a broader area.

The term "VSMTF", or Visual Strehl Ratio based on the Modulation Transfer Function, refers to a metric used to evaluate the quality of an optical image, particularly in the context of assessing human vision and optical correction methods. This metric integrates the traditional concept of the Strehl Ratio, which is a measure of peak image sharpness, with the Modulation Transfer Function (MTF), a standard measure of an optical system's ability to transfer contrast from the object to the image at different spatial frequencies.

The term "VSOTF", or Visual Strehl Ratio based on the Optical Transfer Function, refers to a metric used to evaluate the quality of an optical image, with a specific focus on the human visual system and its correction. This metric combines the principles of the Strehl Ratio, which assesses peak image sharpness, with the Optical Transfer Function (OTF), a comprehensive measure that includes both the Modulation Transfer Function (MTF) and the Phase Transfer Function (PTF).

## Description

A significant advancement in objective refractive error determination has been the introduction of wavefront aberrometry, which maps the optical imperfections of the eye. However, deriving an optimized visual prescription from this data is complex. Current methods are either inaccurate or computationally very expensive. Current fast methods may require obtaining a prescription for every data point, and then the sum of the refractive corrections is averaged or a statistical representation such as the mode is obtained, to calculate the associated refractive correction or prescription. Other approaches might fit the wavefront with a polynomial expansion and derive the refractive parameters from the polynomial's coefficients. Both these approaches lead to a poor optical quality because they can be highly biased by the presence of outliers or might overrepresent the importance of peripheral locations of the pupil over more central ones. The most accurate methods might involve a nonlinear optimization in a multidimensional space that often converges towards a local minima, which usually means that it requires a starting point close to the final solution. Alternatively, a brute force approach might be employed to scan all the possible prescription combinations over a multidimensional space, which results in a very time-consuming task.

Therefore, there is a pressing need for a method to obtain an optimized objective visual prescription that excels in image quality metrics and represents subjective visual quality realistically. This method should minimize the influence of outliers, not rely on proximity to the final solution for its starting point to find an objective solution, which would make it non-robust, and, most importantly, be computationally efficient, allowing for its application during a standard ophthalmological visit to provide immediate and accurate prescriptions.

The present invention offers a solution to all these drawbacks. This solution is based on the clustering of a curvature map that represents a light wavefront that has passed through an optical system, and does it before computing the refractive correction needed. After the clustering, the refractive correction is calculated only for the number of clusters, and the best one is selected. This allows for a very efficient, quick, computationally lightweight determination of a prescription (refractive correction), while performing outstandingly good according to objective image quality metrics. Clustering also prevents the obtention of a statistically representative curvature value that is influenced by outliers, or by peripheral parts of the pupil, solving this way all the current challenges in the field, and completely changing the paradigm of objective optical prescription *in situ* in ophthalmological clinics.

The algorithm herein described, for example, may depart from the wavefront slopes measured by an aberrometer, or by a virtual eye model, to create a pupil vergence map, where each point analysed is treated as having its own refractive error. For each point sampled inside the pupil, the aberrometer or virtual eye platform measures the slope in the x and y directions. From these slopes the principal local curvatures can be directly calculated using differential geometry methods, such as gradient calculation, curvature calculation, gaussian curvature, mean curvature and/or Ricci curvature, preferably mean curvature.

The method herein described uses a statistical treatment of the local refractive error to segment the map in clusters of similar value, or low error variation. The clustering step may be achieved by employing one of several clustering algorithms, such as, but not limited to, k-means, DBScan, Hierarchical Clustering and/or Spectral clustering, among others. The rationale is that delimiting a cluster in the pupil where the light has a similar mean refractive error is equivalent to finding a zone that produces a smaller spread of the light, i.e., a more compact point spread function (PSF), with a significant amount of energy to form a sharp image of an object. Identifying k clusters is then equivalent to identifying k possible refractive corrections. This reduces the problem identified before, from a search space in the order of thousands of trials, to k trials, where k is the number of clusters, with typical values ranging from 4 to 6 clusters. The number of clusters obtained after the segmentation is dependent on the spreading of the local refractive errors across the pupil. The more complex the wavefront is, the more zones will be necessary to delimit k zones with a similar mean and low standard deviation, therefore this clustering step is fundamental in order to obtain a statistically representative value of the refractive correction needed in an optical system such as an eye, and, most importantly, by clustering *before* computing the optical prescription, the process is expedited and lightweight.

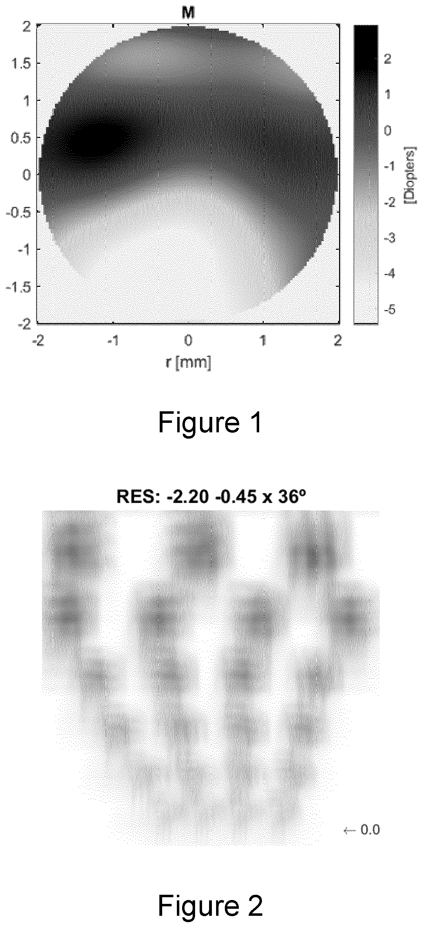

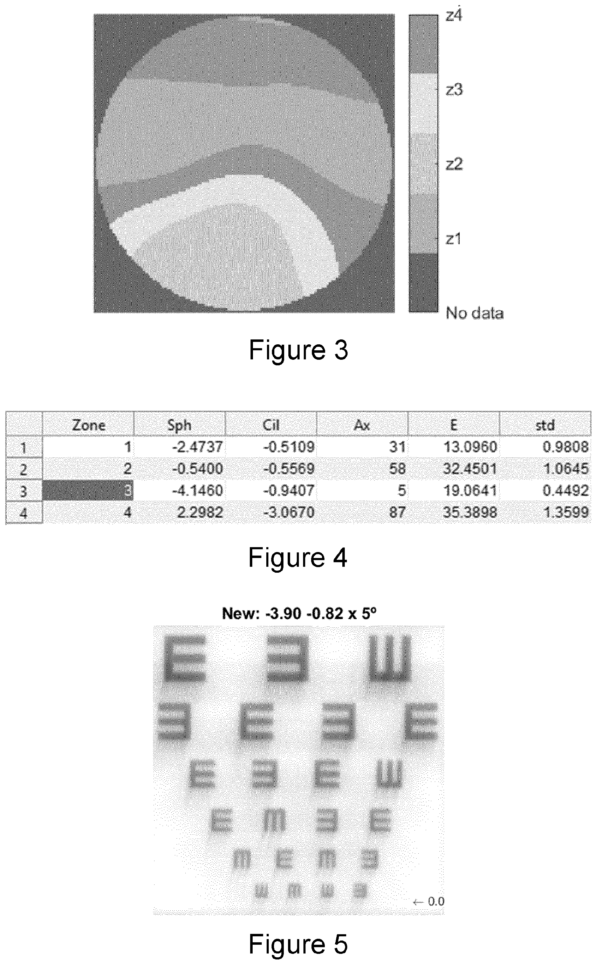

Here we provide examples and figures that back up the above-mentioned results. In example 1 it is shown how, departing from a mean curvature map (figure 1) extracted from data representing a light wavefront passing through a pupil, the current state of the art method to calculate an objective refractive correction yields a blurry and sub-optimal result (figure 2), wherein segmenting the image in clusters (image 3) according to the method of the invention, calculating the objective refractive correction for each cluster (Figure 4), and selecting the best performing one according to a visual quality metric and/or to the least standard deviation yields a much better result (see figure 5), wherein visual impairment is almost unnoticed.

In example 2, it is shown that the method of the invention (figure 12d) provides a prescription with a great visual acuity departing from the mean curvature map shown in figure 7a1, compared to the optimal prescription (figures 12a, b, and c), provided by analysing VSX, VSMTF and VSOTF image quality metrics (figure 9) on an iterated set of 648 prescriptions, i.e., on a set of points representing the whole curvature map, which is a much slower and computationally demanding process. Figure 12d uses the prescription value corresponding to cluster 2 (figure 10), which is selected as providing the best image quality according to the three metrics mentioned before (VSX, VSMTF and VSOTF). A 3D representation of the wavefront error from the mean curvature map shown in figure 7a1 can be seen on figure 6. Figure 8, on the other hand, shows the clusters (figure 8b) obtained after an inertial method was used (figure 8a) to calculate the optimal number of clusters in which the curvature map should be segmented, which in the case of example 2 was 6, as the minimum in the curve depicted in figure 8a suggests.

Therefore, this method of clustering allows to compute the refractive error for each cluster, reducing the number of possible prescriptions to a very limited number, such as below 20, or preferably, below 10, and even more preferably between 4 and 6, allowing for a quick calculation of the respective refractive corrections, or prescriptions, not needing to iterate the calculations for a too big number of possibilities.

Clustering also presents a *priori* unexpected benefits, since by analysing the examples provided it can be deduced that, for instance, when clustering data according to the variation between the data points, if there are few clusters then there are not many points that diverge too much from an average value, therefore the values of one of the cluster will be close to a representative value that yields a good visual acuity. In the other hand, if there is more variability among points, and therefore more clusters, then the most divergent or outlier points will be comprised in certain clusters, but not in the others, thus when calculating the prescription of each of the clusters, and evaluating its image quality, the best results will be obtained on some clusters representative of a prescription that is not affected by these extremely high or low data points. Another unexpected benefit of clustering comes from the fact that if at least part of the pupil correctly resolves an image, the overall result will be much better than an average result over all data points wherein no significative area of the pupil is correctly resolving the image. Similarly, as seen in the images provided in the examples, when segmenting in clusters, the peripheral points of the pupil or diaphragm will be most likely clustered together, since the curved geometry of the pupil is continuous and derivable, and therefore the imperfections will be gradual. This way, the points of the pupil responsible for a better visual acuity will be also mostly comprised in one of the clusters, for which the image quality will be separately evaluated, thus increasing the possibilities of finding an objective prescription that is not affected for an excessive weight of the peripheral parts of the pupil.

This, in turn, allows to obtain a precise and optimized objective refractive prescription quickly and *in situ,* for example, in an ophthalmological centre during a patient's visit. Furthermore, the method of the invention is successful in highly aberrated eyes, where current objective methods fail, and wherein subjective determination is imprecise and relies on input from the patient while manually iterating different combinations, that may not comprise an optimized one, or may stop at a local visual acuity maximum.

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

For the above-mentioned reasons, a first aspect of the invention relates to a computer implemented method for determining a refractive correction for an optical system, preferably wherein the optical system is an eye, more preferably a human eye. The method comprises the following steps:

> a. computing a curvature map from data representing a light wavefront that has passed through the optical system;
>
> b. segmenting the curvature map computed in step (a) into at least two clusters of curvature data points according to the variation between the data points within each cluster;
>
> c. computing the mean curvature of the light wavefront in each cluster from step (b);
>
> d. computing for each cluster the refractive correction associated to the mean curvature of the light wavefront of that cluster as computed in step (c) the; and
>
> e. determining the refractive correction for the optical system as one of the refractive corrections computed in step (d) of the clusters of the curvature map.

Preferably, the method is performed from step a to step e in alphabetical order.

It is noted that the optical system, as referenced above, may encompass a broad range of systems including, but not limited to, human eyes, animal eyes, artificial optical devices, and virtual simulations of optical systems. In the context of the claimed method, an optical system may be any system capable of transmitting or refracting light. Additionally, in some embodiments, the optical system may vary in complexity from a simple lens to a complex arrangement of multiple optical elements. According to some other embodiments of the invention, virtual simulations may include the use of commercially available platforms such as Zemax or OSLO or other software wherein the values that optically characterize an eye are introduced, such as the shape and material of cornea and crystalline/intraocular lens (IOL), and the position of the retina. In some embodiments of the invention, this type of software may be included as a variable in the algorithm/procedure to obtain the best correction or prescription for an eye of a patient, and the use of aberrometers may also be involved.

Hereinafter the optical system may be referred to as an eye, or as an ocular system, therefore the term eye or ocular system also may be applied hereinafter to denote any optical suitable optical system.

Regarding step a) computing a curvature map from data representing a light wavefront that has passed through the optical system, it is noted that the curvature map, generated from data representing a light wavefront that has passed through the optical system, can take various forms. For example, it may be a map of local curvature in each data point. Preferably, the curvature map is represented in dioptres, that show the deviations in the wavefront of light as it passes through the optical system. In some embodiments, the curvature map may be represented using colour code that accounts for the deviation or aberrations, or using Zernike polynomials, or a combination of both. Preferably, the curvature map is represented as mean curvature maps (M), Cartesian astigmatism maps (J0), and/or oblique astigmatism maps (J45) or any combination thereof.

Preferably, from light wavefront data, the mean curvature map (M) is obtained by calculating the curvatures from the gradient data (slope measurements) of a light wavefront, obtained, for example, with an aberrometer, and then calculating the mean curvature at each point as the average of the principal curvature in the x and y orthogonal directions. Regarding the Cartesian astigmatism map (J0), preferably it is obtained by decomposing the light wavefront data into Zernike polynomials and extracting the Zernike coefficients corresponding to horizontal/vertical astigmatism. Similarly, for oblique astigmatism maps (J45), preferably they are obtained by decomposing the light wavefront data into Zernike polynomials and extracting the Zernike coefficients corresponding to oblique astigmatism.

Furthermore, it is noted that the light wavefront, whose curvature is analysed, can be acquired through various means. This may involve the use of an aberrometer or similar devices capable of measuring the light wavefront error. Alternatively, the light wavefront data might be obtained from a virtual 3D model of the optical system or reconstructed from other optical measurements. In some embodiments, a combination of an aberrometer or virtual eye models may be employed.

Preferably, the initial source for the obtention of the curvature map is an ocular wavefront captured by an aberrometer. In some other embodiments, it may be calculated using virtual eye models, modelled using commercially available platforms such as Zemax or OSLO. It is noted that for an aberrometer or a virtual eye model, the entrance pupil diameter is preferably set to match the natural pupil diameter of the eye, such as between 2 and 8 mm, more preferably between 3 and 6 mm, even more preferably 4 mm. In some embodiments, in order to reconstruct the wavefront, a matrix comprising Zernike coefficients may be employed, preferably coefficients up to between 2 and 20 orders, more preferably coefficients up to between 5 and 15 orders, even more preferably coefficients up to the tenth order. Said matrix comprising Zernike coefficients preferably comprises a number of columns and rows in between 10 and 1000 each, more preferably in between 100 and 300 each, even more preferably the matrix has 128 rows and 128 columns.

In some embodiments, in order to recreate the Stiles-Crawford effect, which is a phenomenon in human vision where the effectiveness of light in producing a visual response decreases towards the edge of the pupil, compared to when it enters near the centre in the wavefront, an amplitude function may be employed for the light wavefront. In some embodiments such amplitude function may be, but is not limited to, a Gaussian, Lorentzian, exponential, sigmoid, rectangular or Bessel function, wherein preferably the amplitude function is described as a gaussian amplitude mask. If a gaussian amplitude mask is employed, the apodization factor (a specific value used to adjust how sharply the amplitude decreases from the centre to the periphery) may comprise values between 0.001 and 0.5, more preferably between 0.01 and 0.2, even more preferably the apodization factor has a value of 0.05. It is noted that the equivalent of the apodization factor may be employed for other amplitude functions different than a Gaussian.

A preferred example of a gaussian function used to fit Stiles-Crawford effect data sets may be the following:

$$\eta = \eta_{max} \cdot 10^{-ap(r-c)^2}$$

Wherein, $\eta$ is a measure of sensitivity at a given pupil-entry position, (r - c) is a measure of the distance in the entrance pupil of the eye (in millimetres) from the location of the peak of sensitivity ($\eta_{max}$), and ap is the shape or apodization factor (related to peakedness or curvature and hence the width or spread of the curve). Hence, parameter ap represents the Stiles-Crawford apodization factor, which for a typical eye is in the order of 0.05.

In some embodiments, to represent the light transmission through the pupil of an eye, a mathematical expression such as a pupil function may be employed. Preferably, said pupil function may be defined as the complex exponential dependent on the amplitude function chosen, for a wavelength preferably in the visible range, said wavelength more preferably between 450 and 900 nm, even more preferably between 500 and 700 nm, even more preferably the wavelength has a value of 550 nm. Alternatives to the complex exponential dependent on the amplitude function for the pupil function comprise, but are not limited to: Binary pupil function, apodized pupil function, zonal or modal wavefront representations, phase-only pupil functions or customized pupil function for specific aberrations. The pupil function may help to incorporate the effects of both amplitude modulation (due to the Stiles-Crawford Effect) and phase modulation (due to wavefront aberrations) as light passes through the pupil. The pupil function may influence the calculation of the Point Spread Function (PSF) and the Optical Transfer Function (OTF), which in some embodiments may be used to calculate the image quality metrics (VSX, VSMTF, VSOTF).

In some embodiments, in order to calculate said point spread function (PSF) and its representation in the Fourier domain, which corresponds to the optical transfer function (OTF), preferably Fourier optics methods are be employed, such as, but not limited to, the Fast Fourier Transform (FFT). Alternatively, in some embodiments, the Discrete Fourier Transform (DFT) may be used, particularly for non-uniform or irregularly sampled wavefronts. In some other embodiments, convolution methods may be applied, where the PSF is directly computed by convolving the pupil function with a point source. Additionally, for the OTF, the autocorrelation of the PSF may be computed, or direct methods such as the Angular Spectrum Method may be used to model the18ropagateion of the wavefront through the optical system. Besides, ray-tracing algorithms may be employed for a more geometric perspective on PSF and OTF calculation, which is especially useful in systems with significant aberrations or non-paraxial conditions.

Regarding step b), segmenting the curvature map computed in step (a) into at least two clusters of curvature data points according to the variation between the data points within each cluster, it is noted that the variation between the data points within each cluster may be quantified using various mathematical methods. These may include, but are not limited to, standard deviation, variance, range, or interquartile range. Preferably the variation between data points is quantified using the standard deviation. It is further noted that the segmentation of the curvature map into clusters may be achieved using a variety of methods. These methods may include, but are not limited to, k-means clustering, inertia method, spectral clustering, hierarchical clustering, density-based spatial clustering of applications with noise (DBSCAN), and/or Gaussian mixture models or any combination thereof, preferably the segmentation is achieved through k-means methods. Each method offers a unique approach to grouping curvature data points, which can be selected based on the specific requirements of the optical system under examination, or be used at different stages of the method. Alternatively, in some embodiments, the clustering may be obtained by increasing the number of clustered zones until the standard deviation inside each zone falls below a value that is sufficient to guarantee good image quality inside that zone. In some other embodiments, the number of clusters may be predefined according to an inertia method. Therefore, for the identification of the cluster with the potential to form the best image of a point, different mathematical approaches may be employed, such as the above-mentioned inertia method or, for instance, a weighted average between the standard deviation of the local refractive error and the area of the zone itself, which will be proportional to the light spreading and power created by each individual zone. Other possible clustering approaches may use physical optics principles to calculate PSFs (Point Spread Functions), optical transfer functions or other metrics of contrast for each possible refractive correction. Alternatively, clustering may be based on other characteristics of the data points, such as their geometric proximity, intensity, distance, or other statistical properties. Alternatively, the clustering may be related to the geometry of the pupil, and it may divide areas which provide different contributions to the overall visual acuity.

Regarding step c) computing the mean curvature of the light wavefront in each cluster from step (b), it is noted that the mean curvature in each cluster may be determined through several statistical techniques. These techniques comprise, but are not limited to, the arithmetic mean, which involves summing the curvature values of the data points in each cluster and then dividing by the number of data points, or a weighted mean, where each curvature value is multiplied by a predetermined weight based on certain criteria before summing and dividing by the sum of the weights. Preferably, a mean, or average, of the curvature data points in each cluster is computed. Furthermore, other methods like the median, which identifies the middle value in a sorted list of curvature values, or a trimmed mean, which excludes outliers or the extreme values in each cluster before calculating the average, may also be applied to compute the mean curvature. In some other embodiments the mode, geometric mean, harmonic mean, quartiles and or percentiles may be employed as statistical techniques.

Regarding step d) computing for each cluster the refractive correction associated to the mean curvature of the light wavefront of such the cluster as computed in step the, it is noted that the local curvature maps are preferably decomposed on at least one or more of the three refractive correction components (spherical aberration S, Coma-like horizontal aberrations C0 and coma-like oblique aberrations C45), more preferably in the three of them. Preferably, the refractive correction components S, C0 and C45 are calculated from the Laplacian of the wavefront, which may be obtained through the functions described above, through these equations:

$$S = -\frac{1}{2}\left(W_{XX}''(x,y) + W_{YY}''(x,y)\right)$$

$$C_0 = -\frac{1}{2}\left(W_{XX}''(x,y) - W_{YY}''(x,y)\right)$$

$$C_{45} = -W_{XY}''(x,y)$$

Wherein (x, y) refer to the coordinates of the surface points that conform the surface of the wavefront, and W' is a 2x2 symmetric matrix representing the wavefront curvature of an infinitesimal conic wavefront.

In some other embodiments, the refractive correction may be calculated in terms of the Sphere, Cylinder and Axis, or any other type of prescription. It is noted also that a skilled person may know how to convert a refractive correction into a different refractive correction notation.

According to some other embodiments of the invention, alternatives to calculating said refractive correction components from the Laplacian of the wavefront comprise, but are not limited to, direct fitting to wavefront data, ray tracing algorithms or gradient-based methods.

It is noted that in some other embodiments, the curvature map decomposition may be obtained through Zernike polynomial coefficients, seidel aberrations, or higher order aberrations, among other possible curvature map decompositions. It is also noted that the curvature maps in some embodiments may be calculated in terms of the Mean curvature (M), cartesian astigmatism (J0) and/or oblique astigmatism (J45).

It is further noted that the refractive correction computed for each cluster, and subsequently for the optical system, may correspond to various types of optical corrections. These corrections may relate to prescriptions for glasses or contact lenses, adjustments in the settings of a laser eye surgery apparatus, or parameters for designing custom optical elements. The refractive correction obtained may also be used in the calibration of optical instruments or in the simulation of optical systems. Therefore, the refractive correction may be a prescription for, but not comprised to, a visual or eyes corrector, such as glasses, contact lenses, intraocular lenses or any type of corrective eye surgery, such as LASIK (Laser-Assisted In Situ Keratomileusis), PRK (Photorefractive Keratectomy), LASEK (Laser Epithelial Keratomileusis) or RLE (Refractive Lens Exchange).

In some embodiments, image quality metrics such as, but not limited to, VSX (Visual Strehl ratio based on the extended area), VSMTF (Visual Strehl ratio based on the modulation transfer function), VSOTF (Visual Strehl ratio that is based on the optical transfer function)., RMS (Root Mean Square), EW (Edge Width), SM (Sharpness Metric), HWHH (Half Width at Half Height), CW (Contrast Weighting), NS (Noise Suppression), LIB (Local Image Brightness), STD (Standrad Deviation), and/or ENT (Entropy) may be employed. Preferably, said image quality metrics are be based on sphero-cylindrical lens obtained from the curvature maps, such as VSX, VSMTF or VSOTF, wherein VSX may be calculated according to the following equation:

$$VSX = \frac{\int_{psf} PSF(x,y)N(x,y)dxdy}{\int_{psf} PSF_{DL}(x,y)N(x,y)dxdy}$$

wherein N(x,y) is a bivariate neural weighting function equal to the inverse Fourier transform of the neural contrast sensitivity function for interference fringes, PSF is the point spread function and (x, y) refer to the coordinates of the surface points that conform the surface of the wavefront.

Advantageously, the method of clustering as described in the claim, for determining a refractive correction, offers significant computational efficiency. By reducing the search space for the best possible prescription from thousands of trials to k trials, where k is in the order of 2 to 20, the process becomes less computationally demanding. Traditional methods that require statistical treatment of every data point to obtain representative values like mean or mode are inherently more resource-intensive. This efficiency in computation is further enhanced by limiting the number of possible prescriptions to a manageable range, such as below 20, preferably below 10, and even more preferably between 4 and 6. This limitation allows for the quick calculation of respective refractive corrections or prescriptions without the need to iterate calculations for an excessively large number of possibilities. It is noted that alternative methods may also be quick, as explained above, due to obtaining a mode or an average of the refractive errors, but these methods fail under conditions such as in highly aberrated eyes, or even in the presence of normal amounts of spherical aberration, whereas the method of the invention provides great visual acuity also in these conditions, as shown in the examples.

Furthermore, clustering presents additional, initially unexpected benefits. For instance, when clustering data according to variation between data points, fewer clusters indicate minimal deviation from an average value. In such scenarios, the values within a cluster are likely to be close to a representative value that yields good visual acuity. Conversely, in cases with more clusters, divergent or outlier points are segregated into specific clusters, thereby not affecting the others. Consequently, when calculating the prescription for each cluster and evaluating its image quality, the best results are likely to be uninfluenced by extreme data points. This approach ensures that the chosen prescription optimally represents the needs of the optical system without being skewed by anomalies.

Another significant benefit arises from the geometrical properties of the pupil or diaphragm. Given the continuous and derivable curvature of the pupil, peripheral points are likely to be clustered together, thereby separating the central pupil points, which generally contribute to a better visual acuity, into their own clusters. This segregation means that when evaluating image quality for each cluster, some clusters will represent the areas of the pupil with more similar data points, and therefore they may provide a better image quality when the eye is corrected according to the prescription obtained from said clusters. Such an approach increases the likelihood of identifying an objective prescription that is not adversely affected by excessive emphasis on areas of the pupil with extreme values.

Consequently, this method facilitates the quick and precise determination of an optimized objective eye corrector prescription *in situ,* such as in an ophthalmological centre during a patient's visit. This capability represents a significant advancement over current practices, where objective prescription determination relies on lengthy and demanding calculations, and subjective determination depends on patient feedback while manually iterating through different combinations. The proposed method, therefore, not only streamlines the prescription process but also potentially reaches an optimized prescription that might not be attainable through conventional subjective methods or may otherwise be overlooked in favour of a local visual acuity maximum.

In a preferred embodiment of the method of the first aspect of the invention, the curvature map is segmented into at most 20 clusters. It is noted that cluster segmentation happens preferably in step b).

It is noted that even though it is specified up to 20 clusters, alternative embodiments may consider varying the number of clusters based on specific requirements or constraints of the optical system being analyzed. For example, in simpler systems, fewer clusters might be sufficient, whereas more complex systems may benefit from a number closer to the maximum of 20.

It is also noted that in the experimental section we provide evidence that this number of clusters is more than enough to yield a visual quality that is nearly indistinguishable from the best results obtained from much higher numbers of prescription iterations, such as 648 (see example 2).

Advantageously, limiting the number of clusters to at most 20, enables the execution of the method within the time constraints of an ophthalmologic visit and it allows for real-time optimization of prescriptions based on the segmented curvature data. This feature ensures that the method is not only resource-efficient, making it suitable for use during a patient's visit, but also maintains a high standard of accuracy and visual quality in the refractive corrections determined.

In a more preferred embodiment of the method of the first aspect of the invention, the curvature map is segmented into at most 10 clusters. Preferably, the curvature map is segmented in between 4 and 6 clusters, both values inclusive. It is noted that cluster segmentation happens preferably in step b) of the method.

It is noted that the further reduction of the maximum number of clusters to 10 comes with an increased efficiency and speed in the method for determining refractive correction. This reduction in clusters is significant as it further streamlines the computational process without compromising the output quality of the refractive correction, as it has been demonstrated in example 2.

It is also noted that the preferred range of 4 to 6 clusters represents an optimal balance between computational efficiency and the quality of the refractive correction. This range is based on empirical evidence (see examples) indicating that such a number of clusters is generally sufficient to achieve a high-quality result. The visual quality obtained with the evaluation of 6 clusters, as shown in figure 12, is apparently as good as that achieved with a much larger number of prescription iterations, such as 648, as shown in example 2. Similarly for example 1, wherein the best prescription found based on 4 clusters already provide an excellent visual acuity.

In alternative embodiments, the specific number of clusters within this range may be adapted based on the complexity of the optical system under analysis or specific requirements of the application. For example, more complex systems might benefit from using the higher end of the range (10 clusters), while simpler systems may achieve optimal results with only 3 clusters.

Advantageously, specifying a range of up to 10 clusters, preferably between 4 and 6, for the segmentation in clusters of the method, results in a highly efficient and rapid computation process. This efficiency is particularly beneficial in clinical settings wherein time is a critical factor. Moreover, the method maintains a high standard of accuracy and visual quality in the refractive corrections, despite the reduced number of clusters. The ability to achieve near-optimal results with a significantly lower number of clusters demonstrates the method's robustness and its suitability for various applications where both efficiency and quality are important, being able to change the paradigm of obtaining a visual corrector prescription *in situ* in an ophthalmological clinic in only one visit.

In another preferred embodiment of the method of the first aspect of the invention, the curvature map is segmented into a predefined number of clusters. It is noted that cluster segmentation happens preferably in step b) of the method.

It is noted that predefining the number of clusters may lead to faster calculations as the method bypasses the need to determine the optimal number of clusters dynamically. It is further noted that the predefined number of clusters can be determined based on a variety of factors. One approach may involve statistical analysis of previous calculations to identify a number that frequently results in high-quality refractive corrections. Alternatively, the predefined number could be based on the type of eye imperfection being corrected, where different imperfections may have varying optimal cluster counts. Another approach might involve setting a number that is sufficiently high to ensure a high probability of finding an optimized prescription within the provided clusters.

It is noted that the segmentation of the curvature map into a predefined number of clusters introduces a methodological variation that may offer several advantages. Predefining the number of clusters may therefore lead to faster calculations as the method bypasses the need to determine the optimal number of clusters dynamically.

In addition, predefining the number of clusters may facilitate a comparative analysis of prescriptions derived from different cluster sizes. For example, the method could involve evaluating the image quality associated with prescriptions from 4, 5, and 6 clusters altogether and selecting the one that offers the best visual outcome.

Advantageously, the use of a predefined number of clusters in the method simplifies the computational process and can lead to quicker determination of refractive corrections. Furthermore, the ability to compare results from different predefined cluster numbers enables the method to achieve a high level of accuracy and customization in refractive correction, ensuring that the best possible outcome is selected for each individual case. This aspect of the method combines efficiency with precision, making it a valuable tool in ophthalmic diagnostics and treatment planning.

In a preferred embodiment of the method of the first aspect of the invention, the number of clusters is determined using any of the following methods or any of their combinations: k-means method, inertia method, DBScan, Hierarchical Clustering, and spectral clustering. It is noted that cluster segmentation happens preferably in step b) of the method.

It is noted that from the methods above-mentioned, k-means involves partitioning data into K clusters, minimizing variance within each cluster. The inertia method (see figure 8a), that in some embodiments may be coupled with the k-means method (see figure 8b), calculates the sum of squared distances within clusters to determine the optimal number of clusters, that is, the number K of clusters over which it may be performed the K-means clustering. DBScan groups points based on density, effectively handling noise and identifying arbitrarily shaped clusters, Hierarchical Clustering creates a tree of clusters, and Spectral Clustering uses eigenvectors of a similarity matrix for dimensionality reduction and clustering. Each of these methods has unique characteristics and may be chosen alone or in combination based on the specific requirements of the optical system and the nature of the data being analysed.

It is noted that the skilled person in the field of computational optics and data analysis may identify alternative clustering methods that could be equivalent to those mentioned above, depending on the specific application and data characteristics. Some examples of such alternative methods, but not limited to, may include Gaussian Mixture Models, which assume data clusters follow a Gaussian distribution, Mean Shift Clustering, known for its ability to discover clusters without a pre-specified number, Affinity Propagation, which creates clusters by sending messages between data pairs, and/or Fuzzy C-Means, where data points can belong to more than one cluster, creating a fuzzy partition.

In some embodiments, these methods may be combined or modified to better suit the specific characteristics of the data or the requirements of the optical system being analysed. For example, a hybrid approach using both k-means and DBScan could leverage the strengths of both methods, potentially improving the accuracy of the segmentation, or using k-means and the inertia method to find the optimal K number of clusters.

Advantageously, the use of these advanced clustering methods allows for a more detailed and accurate clustering and determination of the number of clusters, directly impacting the quality of the refractive correction. This approach ensures that the segmentation is closely tailored to the characteristics of the light wavefront data, leading to a more precise and effective refractive correction. The flexibility to use any of these methods or their combinations enables the method to adapt to a wide range of scenarios and data types, further enhancing its applicability and effectiveness in diverse optical systems.

In a preferred embodiment of the method of the first aspect of the invention, the at least two clusters are segmented according to a predefined maximum standard deviation between the data points within each cluster, preferably wherein the maximum standard deviation is 0.5 dioptres (D), more preferably wherein the maximum standard deviation is 0.4 dioptres, even more preferably wherein the maximum standard deviation is 0.25 dioptres.

It is noted that all values provided may present a $\pm 25 \%$ variation, preferably a $\pm 10 \%$, more preferably a $\pm 5\%$ variation.

This approach permits setting a uniformity of refractive errors within each cluster, allowing for a more precise and tailored refractive correction. The specification of a maximum standard deviation, preferably 0.5 dioptres (D), more preferably 0.4, and even more preferably 0.25 dioptres (D) as a segmentation criterion allows for a more uniform distribution of refractive errors within each cluster. This parameter ensures that each cluster represents a specific range of refractive errors with limited variation, which may provide an alternative to accurately assessing and correcting these errors, based on this statistical distribution.

It is also noted that this method of segmentation based on standard deviation, for irregular corneas that present a higher variation in refractive errors may result in a greater number of clusters, providing a broader range of alternatives for finding the most suitable prescription. Conversely, for more regular corneas with less variation in refractive errors, fewer clusters may be required, thereby simplifying the computational process and resolving an optimized prescription quicker.

Advantageously, the use of a predefined maximum standard deviation for cluster segmentation enhances the method's ability to adapt to the individual characteristics of the optical system, particularly the cornea pupillary area. This approach ensures that the method is not only efficient but also highly effective in identifying the most appropriate refractive correction for a wide range of corneal conditions. By catering to the specific needs of different corneal profiles, the method demonstrates a high level of precision and customization in refractive correction.

In a preferred embodiment of the method of the first aspect of the invention, the curvature map comprises one or more of the following: a mean curvature map (M), a cartesian astigmatism map (J0) and/or oblique astigmatism map (J45), wherein preferably the curvature map comprises the mean curvature map (M).

It is noted that the mean curvature map (M) generally represents the average curvature of the cornea, providing a general overview of its refractive characteristics. This map is particularly useful for identifying overall refractive errors and is preferred for its simplicity and effectiveness in many applications. The cartesian astigmatism map (J0) focuses on representing astigmatism along the principal meridians, typically the horizontal and vertical axes. This map is valuable for analysing and correcting astigmatism that aligns with these axes, offering a more detailed view of this specific type of refractive error. The oblique astigmatism map (J45), on the other hand, represents astigmatism at oblique angles, specifically 45 degrees and 135 degrees. This map is used for diagnosing and addressing astigmatic errors that occur along these oblique meridians, which are not captured by the J0 map.

It is further noted that while in some embodiments the mean curvature map (M) (see figures 1 and 6a1) is preferred due to its general applicability and effectiveness, in some other embodiments the skilled person may employ equivalent alternatives such as the J0 (see figure 6b1) or J45 (see figure 6c1) map, or a combination of these maps, to gain a more comprehensive understanding of the cornea's refractive properties. The choice of map or combination of maps may be based on the specific characteristics of the patient's cornea, statistical information of previous patients, and/or the type of refractive error being corrected.

Advantageously, the flexibility to use different types of curvature maps or their combinations in the method of the invention allows for a tailored approach to refractive correction. This adaptability ensures that the method can effectively address a wide range of refractive errors, from general curvature issues to specific types of astigmatism, enhancing its applicability and effectiveness in personalized ophthalmic diagnostics and treatment planning.

In a preferred embodiment of the method of the first aspect of the invention, the refractive correction is determined as the mean refractive correction of the cluster of the curvature map that yields the best image quality according to a predesignated image quality metric.

It is also noted that the skilled person may utilize various metrics for assessing image quality, such as, for instance, VSX, VSMTF, etc. Alternatives might include metrics based on wavefront error, point spread function analysis, or contrast sensitivity. The choice of metric can depend on the specific requirements of the optical system or the patient's eye, the nature of the refractive errors, and the intended application of the method.

Additionally, in some embodiments the choice of the best prescription may be subjective, either by a user evaluating personally the image simulations after correcting refractive errors according to the prescription of each cluster, or by the patient selecting which prescription provides the best visual acuity. However, it is noted that preferably this process is automated using image quality metrics as mentioned above. Automation ensures consistency, objectivity, and efficiency in the selection process, particularly important in clinical settings where time and accuracy are crucial, and also allows to try on prescriptions that may need custom manufacture.

Advantageously, employing image quality metrics for determining the best refractive correction enhances the method's effectiveness in providing high-quality visual outcomes. This approach allows for an objective evaluation of the potential visual improvements offered by each prescription, ensuring that the selected refractive correction maximally benefits the patient's visual acuity. The flexibility to choose between various image quality metrics or to incorporate subjective selection processes further enhances the method's adaptability and its personalized patient care.

According to a preferred embodiment of the invention, the refractive correction is determined as the mean refractive correction of the cluster with the largest area and/or lowest variation between the data points within each cluster.

It is noted that this approach leverages the statistical properties of the cluster to identify an optimum prescription or refractive error correction.

The emphasis on the largest cluster area is significant as it relates to the largest pupillary area, meaning it encompasses more data points that may play a bigger role representing the overall patient's vision. This approach ensures that the chosen refractive correction addresses the most significant portion of the cornea impacting the patient's vision.

Similarly, selecting the cluster with the lowest variation between data points within the cluster is a valuable approach in combination with the cluster with the largest area, or may also be a valuable criterion by itself. This criterion indicates that the refractive error across this cluster is relatively uniform. Therefore, a prescription correcting this cluster's refractive error is likely to be effective over a large area of the cornea. Thus, this prescription may approximate an overall value providing good visual acuity for the patient.

Advantageously, this method of determining refractive correction offers a balanced approach that considers both the extent of the affected area (as indicated by the cluster size) and the uniformity of the refractive error within that area (as indicated by the low variation in data points). This dual consideration, alone or in combination, depending on the case, ensures that the selected prescription is not only representative of the predominant refractive error but may also, or instead, be consistently effective across a significant portion of the cornea. Such an approach enhances the likelihood of achieving a comprehensive and effective refractive correction, tailored to the patient's specific optical characteristics.

In a more preferred embodiment of the first aspect of the invention, the best refractive correction is determined according to at least one image quality metric.

It is noted that determining the best refractive correction based on at least one image quality metric emphasizes an objective approach, in contrast to subjective methods that rely on the opinions of the patient or the clinician. By employing quantifiable and standardized image quality metrics, the method ensures a robust and reproducible process for determining refractive correction.

The objectivity of using image quality metrics removes variability and potential bias inherent in subjective assessments. This approach is especially valuable in clinical settings where consistency and accuracy are important. This method is automatic and quick, and enhances the efficiency of the refractive correction process, making it highly suitable for scenarios where time is an important factor.

Moreover, the method's may rely on statistical data and standardized metrics for continuous improvement and adaptation. It is further noted that the skilled person may adopt new advancements in image quality metrics and newer definitions, or incorporate entirely new metrics into the method.

Advantageously, the objective and automated nature of this method, combined with its adaptability to new advancements in image quality metrics, ensures that it remains a cutting-edge solution for refractive correction. This approach not only provides high-quality, reliable outcomes but also positions the method to evolve alongside future innovations in ophthalmic diagnostics and treatment.

According to an even more preferred embodiment of the method of the first aspect of the invention, the at least one image quality metric is based on common Fourier methods, such as the integration of contrast and phase obtained from the optical transfer function, or from the weighted energy of the point spread function. Preferably, the at least one image quality metric comprises one or more of the following metrics: Visual Strehl ratio based on the extended area (VSX), Visual Strehl ratio based on the modulation transfer function (VSMTF), Visual Strehl ratio that is based on the optical transfer function (VSOTF).

It is noted that these metrics, VSX, VSMTF, and VSOTF, each offer a unique perspective on assessing visual quality and different embodiments of the invention may employ one or more alone or in combination. The VSX metric focuses on a broader area of the eye's wavefront error, providing a comprehensive view of visual quality. The VSMTF metric evaluates the modulation transfer function, which relates to how well an optical system can reproduce (or transfer) various levels of detail from the object to the image. The VSOTF metric, on the other hand, is based on the optical transfer function, assessing the overall quality of an image as it is transferred through the optical system, including factors such as resolution and contrast.

While these metrics are specifically mentioned, it is understood that the skilled person in the field may be aware of or find equivalent metrics for assessing image quality. The field of optical diagnostics and vision science is continually evolving, and new metrics that offer improved accuracy, sensitivity, or relevance to specific clinical scenarios may emerge. Therefore, the method retains flexibility to incorporate any such advancements, ensuring that it remains effective and relevant in the context of ongoing technological and scientific development. This adaptability is a key strength of the method, allowing it to continuously provide accurate and reliable refractive corrections in line with the latest understandings and methodologies in vision science.

It is further noted that these metrics may be the criteria to be used to select which of the k corrections yields the best image quality. Alternatively, in some cases, like in high-aberrated eyes, there may be more than one good optical correction. The method of the invention thus may be used to present to the patient more than one possibility for optical correction, in which case the metric would be the patient's subjective perception.

Advantageously, the use of specific image quality metrics such as VSX, VSMTF, and VSOTF, provides a systematic and objective means of determining the best refractive correction. This approach ensures high precision and consistency in the refractive correction process. The inclusion of these metrics, known for their reliability and accuracy in assessing visual quality, enhances the method's capability to deliver optimal visual outcomes. Furthermore, the method's flexibility to incorporate new or equivalent metrics as they are developed or become known to the skilled person allows for continuous improvement and adaptation to the latest advancements in vision science. This adaptability ensures that the method remains at the forefront of optical diagnostic techniques, offering a robust, future-proof solution for refractive correction. The combination of these factors makes the method highly advantageous for providing precise, reliable, and state-of-the-art solutions in ophthalmic care.

In a preferred embodiment of the method of the first aspect of the invention, the method further comprises determining the sphere, cylinder and axis of such refractive correction.

It is noted that determining the sphere, cylinder, and axis offers an approach for a comprehensive refractive correction. The sphere indicates the degree of nearsightedness or far-sightedness, the cylinder quantifies the degree of astigmatism (if any), and the axis defines the orientation of the astigmatism. This detailed specification allows for precise correction of various refractive errors, encompassing not only the degree of vision impairment but also its specific characteristics. Therefore, in some embodiments, the prescription or refractive error correction may involve determining the sphere, cylinder and axis, alone or in combination. It is further noted that this determination may be performed using algorithms that analyse the data from the curvature map and the selected cluster(s). Furthermore, the algorithm of the invention may calculate the necessary parameters to correct the overall refractive error, including astigmatism, by obtaining the sphere, cylinder and axis, thereby providing a complete and precise vision correction prescription.

Advantageously, the inclusion of these parameters in the method ensures that the refractive correction provided is not just a general improvement of visual acuity but a tailored solution addressing the specific refractive needs of the patient. This comprehensive approach is beneficial for patients with complex refractive errors, as it ensures that all aspects of their vision impairment are accurately addressed. The method's ability to provide a complete prescription enhances its utility and effectiveness in clinical settings, offering a robust and thorough solution for vision correction.

A second aspect of the invention refers to a computer implemented program comprising instructions which, when executed by a processing unit, configures the processing unit to execute the computer-implemented method according to any of the embodiments of the first aspect of the invention.

It is noted that this program may be implemented in various forms and on a range of hardware platforms, demonstrating the versatility of the method. For instance, in some embodiments, the processing unit could be part of a dedicated medical device, specifically designed for ophthalmic diagnostics and treatment. Such a device could be used in clinical settings, providing ophthalmologists with a powerful tool for refractive error analysis and correction.

Alternatively, the program could be implemented on general-purpose computing devices such as desktop computers, laptops, or tablets. This flexibility would allow the method to be accessible in a variety of settings, including smaller clinics or research facilities, without the need for specialized equipment.

The program may also be adapted for cloud computing environments. Thus, in some embodiments the processing and computational tasks may be carried out on remote servers, and the results may be delivered to the user via the internet. This approach could enhance the method's accessibility and scalability, as users would not require powerful local hardware to execute the program, making it even faster and/or more robust and reliable.

Furthermore, the program could be integrated into existing healthcare IT systems, enhancing its utility in a connected healthcare environment. This integration could facilitate data sharing and analysis, improving patient care through a more holistic approach to health data management.

Advantageously, the flexibility in the implementation of this program extends the method's applicability across various platforms and environments. Whether integrated into specialized medical devices, run on personal computing devices, executed in cloud environments, or incorporated into smartphones, the program remains a versatile and powerful tool. This adaptability ensures that the method can be employed in diverse settings and circumstances, thereby broadening its impact and utility in the field of ophthalmology and beyond.

A third aspect of the invention refers to a system for determining a refractive correction, the system comprising: a processing unit and a memory unit, wherein the memory unit comprises a set of instructions configured to execute the computer-implemented method according to any one of the embodiments of the first aspect of the invention, and wherein the processing unit is configured to execute such instructions from the memory unit.

It is noted that various systems may embody this configuration, each tailored to different use cases and environments. For instance, dedicated ophthalmic diagnostic equipment used in clinical environments can integrate this system, such as for instance in combination with aberrometers, offering precision and reliability tailored for medical use. Such equipment can typically be found in hospitals and specialized eye care clinics.

Alternatively, in some embodiments the system can be implemented on general-purpose computing devices such as desktops and laptops. This approach offers flexibility, making the method accessible in diverse professional settings, including smaller clinics and research facilities. These devices, readily available in many environments, can be easily configured with the necessary software to execute the method.

In some other embodiments the method may also be adapted for portable devices like tablets and smartphones. These devices' versatility and evolving processing capabilities make them interesting candidates, facilitating remote diagnostics and mobile health initiatives. This adaptation may also expand the method's reach, enabling its use in telemedicine and outside traditional clinical settings.

It is noted that in some other embodiments, such as a cloud-based configuration, the processing and memory units may be located on remote servers, with users accessing the system over the internet. This setup provides scalability and ease of access, allowing multiple users to benefit from the method without the need for powerful local hardware. It also enables efficient data management and analysis, enhancing the system's utility in a connected healthcare environment.

Moreover, integrating the system into broader healthcare IT infrastructures, like hospital or clinics networks, or in medical devices such as aberrometers, may enhance operational efficiency and patient care. This integration allows for seamless data sharing and analysis, aligning the method with the broader objectives of healthcare delivery.

Advantageously, the flexible design of the system ensures its adaptability across a wide array of hardware platforms, from specialized medical devices to everyday consumer electronics and cloud-based infrastructures. This versatility is crucial for the system's widespread applicability, enabling efficient and effective execution of the method in various scenarios within the healthcare and research domains. The system's ability to conform to different hardware environments significantly broadens its reach, making it a valuable tool in diverse ophthalmic diagnostic and treatment contexts.

**Examples**

In the following examples, the new method is tested against two different existing methods, to describe the advantages of the idea presented in this document. The first example compares the new clustering method to the Refractive Error Sensing (RES) method described by Navarro (summarized in table 1 of "Navarro, R. (2010). Refractive error sensing from wavefront slopes. Journal of Vision, 10(13)"). The second example compares the new method against the best refractive corrections obtained from optimizing with different image quality metrics. Both examples share the main principles and procedures described in the next steps:

This method could be applied to any ocular wavefront. For these examples the initial source was an ocular wavefront from an eye with an irregular cornea, captured by an aberrometer. The entrance pupil diameter was set to 4 mm to match the natural pupil diameter of this eye. Zernike coefficients up to the tenth order were employed to reconstruct the wavefront W (x, y) in a matrix of 128 rows and 128 columns.

The amplitude function was described as a gaussian amplitude mask to recreate the periphery/central weight produced by the Stiles-Crawford effect (equation (1) in: "Applegate, R. A., & Lakshminarayanan, V. (1993). Parametric representation of Stiles-Crawford functions: normal variation of peak location and directionality. JOSA A, 10(7), 1611-1623"), with a gaussian apodization factor of 0.05. The pupil function was defined as the complex exponential dependent on the amplitude function ($A$), for a wavelength ($\lambda$) = 0.550 $\mu$m. The point spread function (PSF) and its representation in the Fourier domain, the Optical Transfer Function (OTF), was calculated using Fourier optics methods departing from the generalized pupil function $P$:

$$P = A \cdot e^{-iW\frac{2\pi}{\lambda}}$$

$$PSF = |FT(P)^2|$$

$$OTF = FT(PSF)$$

Where $i = \sqrt{-1}$, $FT$ is the Fourier transform and $W$ is the wavefront error expressed in microns.

Subsequently, image quality metrics based on those parameters were calculated such as VSX, VSMTF and VSOTF, as described by Thibos et al (wherein VSX is calculated according to equation A23, VSMTF according to equation A31 and VSOTF according to equation A32 in "Thibos, L. N., Hong, X., Bradley, A., & Applegate, R. A. (2004). Accuracy and precision of objective refraction from wavefront aberrations. Journal of vision, 4(4), 9-9.').

This process was implemented through an iterative computational procedure, that for each different probed combination of sphero-cylindrical lens calculates its correspondent image quality metric value. Finally, those spherical-cylindrical combinations for which the maximum VSX, VSMTF and VSOTF are obtained were saved. Next, two new prescriptions were calculated from the curvature maps of the wavefront, one calculated with the refractive error sensing (RES) method and another with the new clustering method one. In both procedures, the local curvature maps decomposed in its three components (S, C0, C45) were calculated from the Laplacian (second derivatives) of the wavefront as described by Navarro et al. (Equation 7 of Navarro, R. (2010). Refractive error sensing from wavefront slopes. Journal of Vision, 10(13)):

$$S = -\frac{1}{2}\left(W''_{XX}(x,y) + W''_{YY}(x,y)\right)$$

$$C_0 = -\frac{1}{2}\left(W''_{XX}(x,y) - W''_{YY}(x,y)\right)$$

$$C_{45} = -W''_{XY}(x,y)$$

Wherein (x, y) refer to the coordinates of the surface points that conform the surface of the wavefront, and W' is a 2x2 symmetric matrix representing the wavefront curvature of an infinitesimal conic wavefront.

These three maps contain the three components of the local refractive error for each point of the pupil sampled. The application of the RES method consists of representing the distribution of refractive errors across the pupil with a histogram and calculating the general refractive error as the value that minimizes the error in more points, i.e. the mode.

The image simulations were obtained as the convolution of the PSF of the optical system and an image of the Snellen chart. The PSF was resized according to the optotype pixel size, to make the angular size in the last line correspondent to 20/20 visual acuity.

**Example 1**

Materials and methods:

For the first example, the number of clusters was selected so that the standard deviation of the mean local refractive error inside at least one clustered zone would be below 0.50 D. Next, from the k zones identified by the clustering algorithm the average refractive error of all points inside the zone that has a larger area to variance ratio, i.e., the best zone = maximum( area / std²), is selected as the refractive correction that is most likely to provide the best image quality. Different mathematical approaches can be employed such us a weighted average between the standard deviation of the local refractive error and the area of the zone itself, which will be proportional to the light spreading and power created by each individual zone. Other approaches can use physical optics principles to calculate PSFs, OTFs, MTFs or other metrics of contrast for each possible refractive correction, and select the best based on same image quality metric.

The figures supporting example 1 are figures 1, 2, 3, 4 and 5, wherein figure 1 shows an image of the pupil local refractive errors (keratoconic eye), figure 2 shows an image of the result obtained with the method described by Navarro, figure 3 displays an image of the segmentation of the pupil into a number of k zones, figure 4 shows the correspondent mean refractive errors at the plane of the pupil and standard deviation, and figure 5 is an image of the result obtained with the new method (optotype), with the vertex correction distance applied.

Conclusions

The RES method might achieve a good result when the wavefront is not too aberrated. In the case of more complex wavefronts, as in the case of an eye without corneal irregularities, the RES method fails to provide acceptable results. On the other hand, the new clustering method provides better theoretical results, based on objective image quality criteria, for more demanding wavefronts, such as in the case of keratoconic eyes.

**Example 2**

Materials and methods:

In the second example, the Inertia method was used to define the number of clusters used to segment the mean curvature map (M) with the K-means algorithm. As in example 1, each cluster provides a possible prescription, and the selection of the prescription that provides the best image quality is based on the preferred metric. In this case, the second cluster provides the best result for all metrics comparable to the image quality results obtained using the much more computationally expensive iterative method.

Figures supporting example 2 are figures 6, 7, 8, 9, 10, 11 and 12. Wherein figure 6 shows a three-dimensional representation of a wavefront from a keratoconic eye, figure 7 shows a wavefront curvature map for a subject with keratoconus and the histograms of the refractive errors associated (note: M, J0 and J45 are equivalent to the S, C0 and C45). Figure 8 is a representation of the score applying the inertia method to select the number of clusters and the mean curvature map segmented, figure 9 shows an optimized prescription for each image quality metric and their score values, figure 10 displays Sphero-cylindrical prescriptions obtained for each cluster of the segmented mean curvature map and its correspondent score for each image quality metric, figure 11 shows the best sphero-cylindrical prescription selected based on the best performance from all indicators of image quality, and figure 12 shows images of the PSF convolved with an eye chart, for visual comparison of the optimized prescriptions obtained from maximizing the VSX, VSMTF and VSOTF metrics with the best prescription obtained from the new clustering method.

Conclusions

A typical iterative method, with 2376 sphere and cylinder combinations in this case, to search for the best prescription based on the maximization of an image quality metric, provided similar results to the new clustering method. The advantage of the latter is that only 6 possible prescriptions were calculated in this process. In some cases, there might not exist one best refraction correction but multiple. This is another advantage of the new method described in this document, that by immediately reducing the search space to a reasonable number of k trials, makes it possible to directly compare all possible outcomes and allow the clinician to choose between the one that offers the best visual acuity with the lower refractive correction.

**Claims**

1. A computer-implemented method for determining a refractive correction for an optical system, the method comprising the following steps:

   a. computing a curvature map from data representing a light wavefront that has passed through the optical system;
   b. segmenting the curvature map computed in step (a) into at least two clusters of curvature data points according to the variation between the data points within each cluster;
   c. computing the mean curvature of the light wavefront in each cluster from step (b);
   d. computing for each cluster the refractive correction associated to the mean curvature of the light wavefront of the cluster as computed in step (c); and
   e. determining the refractive correction for the optical system as one of the refractive corrections computed in step (d) of the clusters of the curvature map.

2. The computer-implemented method according to claim 1 wherein in step b) the curvature map is segmented into at most 20 clusters.

3. The computer-implemented method according to any one of the previous claims wherein in step b) the curvature map is segmented into at most 10 clusters.

4. The computer-implemented method according to any one of the previous claims wherein in step b) the curvature map is segmented into a predefined number of clusters.

5. The computer-implemented method according to any one of claims 1 to 3, wherein the number of clusters is determined using any of the following methods or any of their combinations: k-means method, inertia method, DBScan, Hierarchical Clustering, and spectral clustering.

6. The computer-implemented method according to any one of claims 1 to 3 and 5, wherein the at least two clusters are segmented according to a predefined maximum standard deviation between the data points within each cluster, preferably wherein the maximum standard deviation is 0.5 dioptres (D).

7. The computer-implemented method according to any one of the previous claims, wherein the curvature map comprises one or more of the following: a mean curvature map (M), a cartesian astigmatism map (J0) and/or oblique astigmatism map (J45), preferably the curvature map comprises the mean curvature map (M).

8. The computer-implemented method according to any one of the previous claims, wherein the optical system represents an eye which suffers from astigmatism, coma, spherical aberration and/or comprises an irregular cornea, an ectasia, and/or represents an eye which has undergone corneal surgery, and wherein the curvature map comprises the mean curvature map (M), the cartesian astigmatism map (J0) and the oblique astigmatism map (J45).

9. The computer-implemented method according to any one of the previous claims wherein the refractive correction is determined as the mean refractive correction of the cluster of the curvature map that yields the best image quality according to a predesignated image quality metric.

10. The computer-implemented method according to any one of the previous claims wherein the refractive correction is determined as the mean refractive correction of the cluster with the largest area and/or lowest variation between the data points within each cluster.

**11.** The computer-implemented method according to claim 9, wherein the best refractive correction is determined according to at least one image quality metric.

**12.** The computer-implemented method according to claim 10, wherein the at least one image quality metric comprises one or more of the following metrics: Visual Strehl ratio based on the extended area (VSX), Visual Strehl ratio based on the modulation transfer function (VSMTF), Visual Strehl ratio that is based on the optical transfer function (VSOTF).

**13.** The computer-implemented method according to any one of the previous claims, further comprising determining the sphere, cylinder and axis of such refractive correction.

**14.** A computer-implemented program comprising instructions which, when executed by a processing unit, configures the processing unit to execute the computer-implemented method according to any one of claims 1 to 12.

**15.** System for determining a refractive correction, the system comprising: a processing unit and a memory unit, wherein the memory unit comprises a set of instructions configured to execute the computer-implemented method according to any one of claims 1 to 13, and wherein the processing unit is configured to execute such instructions from the memory unit.

# Figure 1

RES: -2.20 -0.45 x 36°

← 0.0

# Figure 2

Figure 3

| | Zone | Sph | Cil | Ax | E | std |
|---|---|---|---|---|---|---|
| 1 | 1 | -2.4737 | -0.5109 | 31 | 13.0960 | 0.9808 |
| 2 | 2 | -0.5400 | -0.5569 | 58 | 32.4501 | 1.0645 |
| 3 | 3 | -4.1460 | -0.9407 | 5 | 19.0641 | 0.4492 |
| 4 | 4 | 2.2982 | -3.0670 | 87 | 35.3898 | 1.3599 |

Figure 4

New: -3.90 -0.82 x 5°

Figure 5

Figure 6

Figure 7

a)

Adjusted Inertia for each K

b)

## Figure 8

| Method | Sphere | Cylinder | Axis | M | J0 | J45 | VSX | VSMTF | VSOTF |
|--------|--------|----------|------|-----|-----|-----|-----|-------|-------|
| BestVSX | -3.487 | -4.520 | 3 | -6.386 | 2.697 | 0.254 | 0.086 | 0.097 | 0.124 |
| BestVMTF | -3.334 | -4.023 | 3 | -5.886 | 2.364 | 0.254 | 0.076 | 0.113 | 0.107 |
| BestVOTF | -3.487 | -4.520 | 3 | -6.386 | 2.697 | 0.254 | 0.086 | 0.113 | 0.124 |

## Figure 9

| Cluster | M | J0 | J45 | VSX | VSMTF | VSOTF |
|---------|-----|-----|-----|-----|-------|-------|
| 2 | -6.241 | 2.58 | -0.067 | 0.117 | 0.126 | 0.168 |
| 1 | 0.965 | -1.152 | -0.021 | 0.052 | 0.081 | 0.072 |
| 4 | -4.67 | 2.147 | -0.014 | 0.033 | 0.074 | 0.044 |
| 5 | -0.26 | -0.158 | 0.45 | 0.025 | 0.057 | 0.032 |
| 0 | -3.247 | 1.685 | 0.091 | 0.012 | 0.052 | 0.015 |
| 3 | -1.727 | 0.795 | 0.196 | 0.015 | 0.048 | 0.017 |

## Figure 10

|  | Sphere | Cylinder | Axis | M | J0 | J45 | VSX | VSMTF | VSOTF |
|---|---|---|---|---|---|---|---|---|---|
| **Clustering method** | -3.470 | -4.320 | 179 | -6.241 | 2.580 | -0.067 | 0.117 | 0.126 | 0.168 |

# Figure 11

a)
Best VSX
-3.49 D -4.52 Dc 3°

b)
Best VSMTF
-3.33 D -4.02 Dc 3°

c)
Best VSOTF
-3.49 D -4.52 Dc 3°

d) Clustering
method
-3.47 D -4.32 Dc 179°

# Figure 12

Europäisches
Patentamt

European
Patent Office

Office européen
des brevets

# EUROPEAN SEARCH REPORT

**Application Number**

EP 24 38 2030

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FARIA-RIBEIRO MIGUEL ET AL: "Morphology, topography, and optics of the orthokeratology cornea", JOURNAL OF BIOMEDICAL OPTICS, SPIE, 1000 20TH ST. BELLINGHAM WA 98225-6705 USA, vol. 21, no. 7, 1 July 2016 (2016-07-01), page 75011, XP060072048, ISSN: 1083-3668, DOI: 10.1117/1.JBO.21.7.075011 [retrieved on 2016-07-19] | 1-7, 9-12,14, 15 | INV. A61B3/00 A61B3/10 A61B3/103 |
| Y | * Sections 2.1, 2.2, 2.3 and 3 * <br> * table 1 * <br> * figure 1 * | 8,13 | |
| Y | THIBOS L. N. ET AL: "Accuracy and precision of objective refraction from wavefront aberrations", JOURNAL OF VISION, vol. 4, no. 4, 23 April 2004 (2004-04-23), page 9, XP093130527, US ISSN: 1534-7362, DOI: 10.1167/4.4.9 * Methods page 334 * <br> * page 344 * <br> * page 347 * | 8,13 | |
| A | US 7 926 944 B2 (THOMPSON KEITH P [US] ET AL) 19 April 2011 (2011-04-19) * column 24 * <br> * figure 17 * | 1-15 | |
| A | US 2023/296919 A1 (ZHELEZNYAK LEONARD [US] ET AL) 21 September 2023 (2023-09-21) * paragraphs [0106], [0139], [0140] * <br> * figures 16, 17 * | 1-15 | |

**TECHNICAL FIELDS SEARCHED (IPC)**

A61B
G02C
G16H

The present search report has been drawn up for all claims

| Place of search | Date of completion of the search | Examiner |
|---|---|---|
| Munich | 17 May 2024 | Neumann, Wiebke |

EPO FORM 1503 03.82 (P04C01)

## ANNEX TO THE EUROPEAN SEARCH REPORT
## ON EUROPEAN PATENT APPLICATION NO.

EP 24 38 2030

This annex lists the patent family members relating to the patent documents cited in the above-mentioned European search report.
The members are as contained in the European Patent Office EDP file on
The European Patent Office is in no way liable for these particulars which are merely given for the purpose of information.

17-05-2024

| Patent document cited in search report | | Publication date | Patent family member(s) | | Publication date |
|---|---|---|---|---|---|
| US 7926944 | B2 | 19-04-2011 | US | 2005134799 A1 | 23-06-2005 |
| | | | US | 2010231855 A1 | 16-09-2010 |
| | | | WO | 2004086952 A2 | 14-10-2004 |
| US 2023296919 | A1 | 21-09-2023 | US | 2023296919 A1 | 21-09-2023 |
| | | | WO | 2023177740 A1 | 21-09-2023 |

For more details about this annex : see Official Journal of the European Patent Office, No. 12/82

**REFERENCES CITED IN THE DESCRIPTION**

**Non-patent literature cited in the description**

- **NAVARRO, R.** *Refractive error sensing from wavefront slopes. Journal of Vision*, 2010, vol. 10 (13)
- **APPLEGATE, R. A.** ; **LAKSHMINARAYANAN, V.** Parametric representation of Stiles-Crawford functions: normal variation of peak location and directionality. *JOSA A*, 1993, vol. 10 (7), 1611-1623

- **THIBOS, L. N.** ; **HONG, X.** ; **BRADLEY, A.** ; **APPLEGATE, R. A.** Accuracy and precision of objective refraction from wavefront aberrations. *Journal of vision*, 2004, vol. 4 (4), 9-9
- **NAVARRO, R.** Refractive error sensing from wavefront slopes. *Journal of Vision*, 2010, vol. 10 (13)